# EUROPÄISCHE PATENTSCHRIFT

(11) **EP 0 872 261 B1**
(45) Veröffentlichungstag und Bekanntmachung des Hinweises auf die Patenterteilung: **06.05.2004**
(21) Anmeldenummer: 97250210.8
(22) Anmeldetag: 15.07.1997
(51) Int. Cl.: A61N 1/39, A61N 1/378, H01M 6/50

(54) **Implantierbares medizinisches Mehrfunktionsgerät**
Implantable multi-functional medical device
Appareil médical implantable multi-fonctionnel

(30) Priorität: 16.04.1997 DE 19716969
(43) Veröffentlichungstag der Anmeldung: 21.10.1998
(73) Patentinhaber: LITRONIK Batterietechnologie GmbH & Co., 01796 Pirna (DE)
(72) Erfinder: Pilz, Jürgen Dr.sc., 12307 Berlin (DE); Müller, Jürgen Dr. Ing., 12055 Berlin (DE); Staub, Roland Dipl. Ing., 01819 Berggiesshübel (DE); Fehrmann, Gerd Dipl. Chem., 01796 Pirna (DE)
(74) Vertreter: Eisenführ, Speiser & Partner

(56) Entgegenhaltungen:
- US-A- 5 191 884
- US-A- 5 372 605
- US-A- 5 614 331

## Beschreibung

Die Erfindung betrifft ein implantierbares medizintechnisches Mehrfunktionsgerät - insbesondere ein Herzschrittmacher/Defibrillator-Kombinationsgerät - , bei dem verschiedene Therapiefunktionen unterschiedliche Anforderungen an die Batterie hinsichtlich der Belastbarkeit stellen. Dies kann die verfügbare Stromstärke oder auch die verfügbare Leistung betreffen.

Insbesondere kann für eine erste Funktion über längere Zeiträume ein geringer Strom im µA-Bereich gefordert sein und für eine zweite Funktion bei bestimmten, in unregelmäßigen Abständen auftretenden, Ereignissen ein kurzzeitiger Impulsstrom über mehrere Sekunden im Ampèrebereich.

Energiequellen für derartige Anforderungen sind bekannt, werden heute kommerziell angeboten und in der Humanmedizin eingesetzt. Es handelt sich bei diesen Energiequellen um entsprechende elektrochemische Systeme, die - je nach konstruktivem Aufbau - in der Lage sind, Stromstärken in Bereichen zwischen 10⁻⁶A oder und nahezu 10 A abzugeben.

Das elektrochemische System Lithium/Silber-Vanadium-Oxid ist besonders befähigt, diese Bedingungen zu erfüllen; Einzelheiten sind beschrieben in den US-Patentschriften 4 310 609, US 4 391 729, und US 5 114 811 sowie der europäischen Patentanmeldung EP 0 638 946 A2.

Auch mit dem System Lithium/Vanadiumoxid sind entsprechende Batterien bekanntgeworden; vgl. hierzu die US 3 655 585 und US 3 947 289 sowie Honeywell Power Sources Center [1974], ECOM Final Report 71-0191-F, IV-1-IV-47.

Ein weiteres System mit praktischer Nutzung ist das System Lithium/Manganoxid/Bleioxid/Chromoxid, beschrieben in DE 4 438 784 A1 und Gegenstand der unveröffentlichten europäischen Patentanmeldung EP 95250273.0.

Bekannte Batterien enthalten Metalloxid- und Lithiumelektroden in einem organischen Elektrolyten. Solche elektrochemischen Systeme sind maximal bis 30 mAcm² belastbar. Leistungen in der Größenordnung von 10-W-Impulsen können daher nur durch Batteriekonstruktionen mit hoher Oberfläche erreicht werden. Durch den hohen Anteil passiver Bauelemente in solchen Batterien ist die Energiedichte in Wh/l und Wh/kg relativ niedrig.

Es kommt hinzu, daß alle oben genannten elektrochemischen Systeme den Nachteil besitzen, daß sie auch wegen der Leistungsanforderungen in unterschiedlichen Lastbereichen eine relativ niedrige Energiedichte in Wh/kg und Wh/l besitzen. Das führt dazu, daß entweder Masse und Volumen der Batterie für eine akzeptable Betriebsdauer sehr hoch sind oder kleine und leichte Geräte relativ schnell ausgetauscht werden müssen.

Aus der US 4 134 408 ist eine Anordnung zur Stromversorgung eines implantierbaren Schrittmachers bekannt, die neben der üblichen (Primär-)Batterie Mittel zur zeitweiligen externen Energieversorgung sowie - diesen zugeordnet - eine (Sekundär-)Batterie mit niedrigerer Kapazität umfaßt, die von extern her aufgeladen werden und den Schrittmacher dann jeweils für eine begrenzte Zeit versorgen kann, um die Primärbatterie zu schonen.

In der US-Patentschrift Nr. 5 591 212 ist eine sogenannte Hybrid-Batterieanordnung für einen implantierbaren Impulsgenerator, speziell einen implantierbaren Defibrillator beschrieben, die neben der Batterie einen Kondensator mit hoher Energiedichte aufweist, der selektiv mit der Batterie verbunden wird, um während Betriebsphasen mit hohem Strombedarf die Versorgung der Steuerelektronik des Gerätes sicherzustellen. Eine ähnliche Lösung wurde für einen Herzschrittmacher bereits früher in den US-Patentschriften Nr. 4 416 282 und 4 590 941 beschrieben. Letztere Druckschriften lehren außerdem das Vorsehen einer Notfall-Zusatzbatterie, die im Fall plötzlichen Spannungsabfalls der Hauptbatterie zugeschaltet werden kann.

Das Vorsehen eines teuren und voluminösen Hochleistungskondensators nur zum Zwecke des Strom-Managements (ohne daß er zusätzliche Energie liefern könnte) ist jedoch letztlich ebenso unbefriedigend wie das einer Notbatterie, die als solche weder eine Anpassung an unterschiedlichen Strombedarf noch eine wesentliche Erhöhung der Lebensdauer erlaubt.

In der PCT-Anmeldung WO 94/02202 wird - unter Erwähnung älterer Zwei-Batterien-Anordnungen von Herzschrittmachern - ein implantierbarer Kardioverter/Defibrillator beschrieben, bei dem für die Wahrnehmungsfunktionen einerseits und die Ausgabe von Reizenergie andererseits spezielle Batterien vorgesehen sind, die auf lange Lebensdauer bei sehr geringen Stromstärken einerseits bzw. die Bereitstellung eines hohen Spitzenstroms und extrem niedriger Selbstentladung andererseits optimiert sind. Es wird speziell je ein Paar Lithium-Iod-Batterien und Lithium-Vanadium-Pentoxid-Batterien eingesetzt. Dieses Gerät hat nur eine einzige Therapiefunktion; zudem ist die unflexible Funktionszuordnung der Batterien unbefriedigend.

Aus der US 5,191,884 ist ein Defibrillator bekannt, der eine Schrittmachereinheit aufweist, welche von einer ersten Batterie versorgt wird und ein Hochenergieschocksystem, welches von einer zweiten Batterie versorgt wird.

Aus der US 5,372,605 ist ein implantierbarer cardioverter Defibrillator bekannt, welcher zwei Batterien aufweist und eine Schaltung die im Falle der Erschöpfung einer ersten Batterie oder im Falle von Stromspitzen zusätzlich Strom aus einer zweiten Batterie bezieht.

Der Erfindung liegt die Aufgabe zugrunde, ein implantierbares medizintechnisches Mehrfunktionsgerät bereitzustellen, welches in Bezug auf seine einen geringen Stromverbrauch aufweisene Grundfunktion eine wesentlich erhöhte Betriebsdauer aufweist. Dies soll insbesondere gelten bezogen auf die Masse und/oder das Volumen der eingesetzten Batterieanordnung.

Diese Aufgabe wird gelöst durch ein Mehrfunktionsgerät mit den Merkmalen des Anspruchs 1.

Die Erfindung schließt die Lehre ein, die Aufgaben der Lieferung eines niedrigen (Dauer-)Stroms für eine erste Therapiekomponente und eines hohen (Impuls-)Stroms für eine zweite Therapiekomponente über den größeren Teil der Gerätelebensdauer getrennt durch zwei verschiedene Batteriekomponenten zu erfüllen. Dabei sind die Kapazität und die Leistung der Baugruppen so konzipiert, daß das medizintechnische Gerät über seine Lebensdauer in seinen wesentlichen Funktionen zuverlässig betrieben werden kann. Das für den bei einem derartigen Gerät erforderlichen Energiebedarf insgesamt benötigte Bauvolumen läßt sich auf diese Weise optimieren.

Zu dem Zeitpunkt, zu dem die erste Batteriekomponente für die niedrige Stromstärken erfordernde Funktion soweit erschöpft ist, daß ein unterer Spannungswert unterschritten oder ein oberer Innenwiderstandswert überschritten wird, wird die erste Therapiekomponente selbsttätig auf die zweite Batteriekomponente umgeschaltet. Diese hat bis dahin nur gelegentlich Hochstromimpulse geliefert und ist dafür - normale Einsatzbedingungen unterstellt - in ihrer Kapazität meist überdimensioniert. Ab dem Umschaltzeitpunkt liefert die zweite Batteriekomponente - bevorzugt neben der weiteren Versorgung der zweiten Therapiekomponente (Bereitstellung der kurzzeitigen Hochstromimpulse) - den benötigten Strom für die erste Therapiekomponente (Dauerstrom). Je nach konkreter Ausführung kann sie diese Aufgabe abwechselnd mit der ersten Batterie (speziell in Erholungsphasen für diese) oder allein kontinuierlich erfüllen.

In einer vorteilhaften Ausführung wird durch die Kombination einer ersten Batterie, einer Li/I-Batterie hoher Energiedichte (≥ 700 Wh/l, ≥ 170 Wh/kg) für den Niederstrombereich, mit einer zweiten Batterie, einer Batterie niedriger Energie-, aber hoher Leistungsdichte (≥ 300 Wh/l, ≥ 130 Wh/kg) für den Impulsbetrieb, erreicht, daß für die Betriebszeit von mehreren Jahren beide Batterien in ihren optimal-spezifischen Stromdichte-Bereichen beansprucht werden.

Je nach Einsatzfall kann die Kapazitätsauslegung der Li/I-Batterie so erfolgen, daß ihre Erschöpfung nach 4, 5, 6 oder mehr Jahren erfolgt. Die konkrete Ausführung der Kombination beider Batterien hängt ausschließlich von den Anforderungen des Anwendungsfalles ab. Die Nennspannung der ersten Batterie beträgt vorzugsweise 3 oder 6 Volt.

Wichtig für die zuverlässige und sichere Funktion der Batterieanordnung ist eine elektronische Schaltung, die bis zum Umschaltzeitpunkt gewährleistet, daß jeweils alle Hochstromimpulse nur von der zweiten Batterie übernommen werden und der gesamte Niederstrombedarf von der ersten Batterie abgedeckt wird.

Die Schaltung ist in einer zweckmäßigen Ausführung derart ausgebildet, daß beide Batterien an die beiden Eingänge eines Stromversorgungsteils angeschlossen sind, dessen Ausgang eine Niederstrom-Steuerschaltung versorgt, und daß die zweite Batterie zusätzlich an die Hochstromschaltung angeschlossen ist, die über Steuerleitungen mit der Steuerschaltung verbunden ist. Der Stromversorgungsteil ist aus einem zur ersten Batterie parallel geschalteten Kondensator, einer Spannungsfühler-Vergleichereinheit (Spannungskomparator), einem Logikteil, zwei elektronischen Schaltern und einem Spannungsregler aufgebaut. Beide Eingänge des Stromversorgungsteils für die Steuerschaltung sind bei dieser Ausführung über jeweils einen elektronischen Schalter mit dem Eingang eines Spannungsreglers verbunden, dessen Ausgang gleichzeitig den Ausgang des Stromversorgungsteils darstellt, wobei die elektronischen Schalter von dem mit der ersten Batterie verbundenen Eingang über den Spannungskomparator und einen Logikteil, welche hintereinander angeordnet sind, angesteuert werden.

Die Steuerschaltung besteht insbesondere aus einer Herzschrittmacherschaltung und einer Steuerung für die Hochstromschaltung. Ein Eingang dieser Steuerschaltung ist mit dem Ausgang des Spannungskomparators verbunden.

Die Hochstromschaltung wird von der Steuerschaltung bei Bedarf (beispielsweise für das Aufladen des Schockkondensators bei einem implantierbaren Defibrillator) aktiviert und ist ansonsten ausgeschaltet, d.h., sie entnimmt der zweiten Batterie im Ruhezustand keinen Strom.

Die Steuerschaltung wird in einer ersten Betriebsphase von der ersten Batterie versorgt; der Eingang des Spannungsreglers im Stromversorgungsteil ist über den elektronischen Schalter mit der ersten Batterie verbunden, der zweite elektronische Schalter ist offen. Sinkt die Spannung der ersten Batterie infolge Entladung unter einen batteriespezifisch fest vorgegebenen Wert, schalten der Spannungskomparator und über den Logikteil beide elektronische Schalter um; der Eingang des Spannungsreglers ist nun mit der zweiten Batterie verbunden, und der erste elektronische Schalter ist offen. Die Steuerschaltung wird hierbei von der zweiten Batterie versorgt. Als Umschaltkriterium kann auch ein Anstieg des Innenwiderstandes über einen vorbestimmten Wert benutzt werden, und zur Erfassung kann auch eine Strommeßschaltung dienen. Es kommt dabei auf die Ermittlung einer Meßgröße an, welche das Erreichen einer Restladungsgröße der Batteriekomponente anzeigt, bei deren Unterschreiten diese nicht mehr in der Lage ist, die erste Baugruppe betriebsbereit zu halten.

Der Logikteil enthält im einfachsten Fall lediglich eine Schaltung zur gegenphasigen Ansteuerung der elektronischen Schalter. Nach dem Ansteigen der Spannung der nach Umschaltung auf die zweite Batterie unbelasteten, sich erholenden ersten Batterie kippt die Schaltung in ihren Ausgangszustand zurück, wodurch die Spannung erneut abzufallen beginnt, usw. In diesem Fall wird die Steuerschaltung (d.h. die erste Therapie-Baugruppe) abwechselnd von der ersten und von der zweiten Batterie versorgt. Mit einem bistabilen Element im Logikteil kann dies verhindert werden. Die Steuerschaltung wird dann ab dem Umschaltpunkt allein von der zweiten Batterie versorgt.

Gleichzeitig wird der Steuerschaltung die Information hinsichtlich des Erreichens dieses Umschaltpunktes zugeführt, die bei der ERI (Elective Replacement Indication)-Bestimmung berücksichtigt werden kann.

Bei der erfindungsgemäßen Lösung kann der Ersatz des Implants im Vergleich zu herkömmlichen Lösungen hinausgeschoben werden, da eine - meist noch eine beträchtliche - Reserveenergie bereitgestellt wird.

Zur Bestimmung des entgültigen ERI-Punktes genügt es, den Entladezustand der zweiten Batterie hardware- und/oder softwaremäßig zu überwachen. Der ERI-Punkt ist dann erreicht, wenn auch die zweite Batterie soweit erschöpft ist, daß sie gerade noch die Energie für den zuverlässigen Betrieb des Implantats während einer definierten Zeitspanne (einschließlich einer definierten Anzahl von Maximalenergieschocks bei einem Defibrillator) enthält.

Um auch den hinausgeschobenen ERI-Punkt zu ermitteln, sind bevorzugt vorgesehen: ein weiterer Fühler mit einer weiteren Meßeinrichtung zur Erfassung einer für die Restenergie der zweiten Batteriekomponente unter Berücksichtigung des Strom- und/oder Spannungsbedarfs der ersten Therapiebaugruppe charakteristischen Meßgröße, eine eingangsseitig mit dem weiteren Fühler verbundenen Meßeinrichtung zur Ausgabe eines weiteren Steuersignals, wenn die Restenergie der zweiten Batteriekomponente für den Betrieb der ersten Therapie-Baugruppe mit niedrigem Strombedarf nur noch für einen begrenzten Zeitraum ausreichend ist, sowie eine mit dem Ausgang der weiteren Meßeinrichtung verbundene Telemetrieeinrichtung (24), die ein Telemetriesignal an einen außerhalb des Körpers des Patienten befindlichen Empfänger abgibt, welches von dem weiteren Steuersignal beeinflußt ist.

Wenn das Ausgangssignal der weiteren Meßeinrichtung mit demjenigen der Meßeinrichtung derart zusammengeführt ist, daß das Telemetriesignal sowohl von dem Steuersignal als auch von dem weiteren Steuersignal in unterschiedlicher Weise beeinflußt ist, können die Umschaltpunkte beider Meßeinrichtungen von extern her kontrolliert werden. Der behandelnde Arzt erhält somit bei einer entsprechenden Abfrage mittels eines Programmiergeräts also zuerst eine Mitteilung, wenn die Abschaltung der ersten Batterie erfolgt ist. Je nach Anzahl der vom Patienten benötigten Defibrillationsschocks steht hier noch eine beträchtliche Energiereserve zur Verfügung, welche das Ende der Betriebszeit der Niedrigenergie-Baugruppe noch weit hinausschiebt, so daß auf keinen Fall die Notwendigkeit einer Reimplantation nur aus dem Grunde entsteht, daß die mit einem niedrigen Strom belastbare Batterie ausfällt. Auf das Ende der voraussichtlichen Betriebszeit der zweiten Batterie weist dann ein weiteres - in Kombination übertragenes - Telemetriesignal hin.

Bei Defibrillatoren mit einer zusätzlichen Schrittmacherkomponente weisen die erste und die zweite Batteriet oft unterschiedliche Klemmenspannungen auf. Hierbei ist es dann günstig, wenn ein Spannungswandler vorgesehen ist, der durch das Steuersignal in seinem Übersetzungsverhältnis derart umschaltbar ist, daß es bei Verbindung der ersten Therapie-Baugruppe mit der ersten Batteriekomponente im wesentlichen dem Verhältnis der Klemmenspannung der ersten Batteriekomponente zur Versorgungsspannung der ersten Therapie-Baugruppe und bei bei Verbindung der ersten Therapiebaugruppe mit der zweiten Batteriekomponente im wesentlichen dem Verhältnis der Klemmenspannung der zweiten Batteriekomponente zur Versorgungsspannung der ersten Therapiebaugruppe entspricht. Damit ist sichergestellt, daß nach Umschaltung der Batterie die Niedrigstrom-Baugruppe ohne wesentliche Energieverluste auch mit einer anderen Versorgungsspannung bei im wesentlichen gleichbleibenden Wirkungsgrad betrieben werden kann.

Andere vorteilhafte Weiterbildungen der Erfindung sind in den Unteransprüchen angegeben bzw. werden an einem Ausführungsbeispiel anhand der Figuren näher erläutert. Es zeigen:
Figur 1 ein vereinfachtes Blockschaltbild eines implantierbaren kombinierten Defibrillators/Herzschrittmachers und
Figur 2 ein Schaltbild des Stromversorgungsteils des Gerätes nach Figur 1.

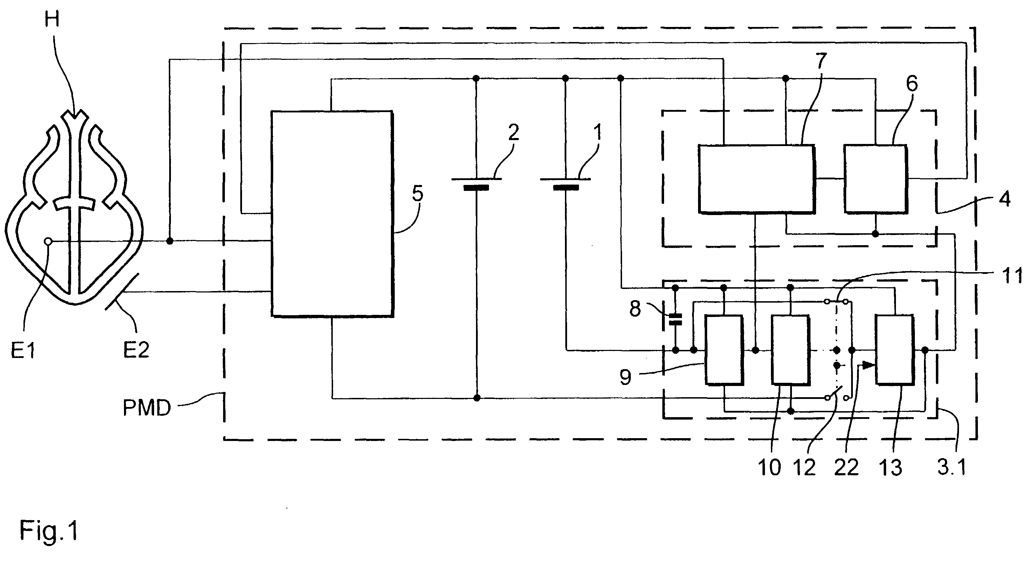

In Fig. 1 sind die für die Erläuterung der Ausführungsform der Erfindung wesentlichen Baugruppen eines implantierbaren kombinierten Schrittmachers/Defibrillators PMD dargestellt; der übrige Aufbau entspricht bekannten Geräten. Als erste Batterie 1 für den Schrittmacher/Defibrillator PMD wird eine 3-V-Li/I-Batterie mit einer Kapazität von 680 mAh bis 2,5 V Entladespannung mit den Abmessungen 12 x 32 x 7 mm bzw. 2,7 cm³ eingesetzt. Als zweite Batterie ist eine 6-V-Li/MnO₂-Batterie 2 mit einer Kapazität von ≥ 350 mAh für den Impulsbetrieb und den Abmessungen 18 x 32 x 12,5 mm bzw. 7,2 cm³ vorgesehen.

Die Li/I-Batterie 1 liefert für mindestens 4,85 Jahre ausreichende Energie für die Steuerschaltung bei einer angenommenen Stromaufnahme von 16 µA. Bis zu diesem Zeitpunkt kann die zweite Batterie 2 die Energie für jährlich mindestens 12 Einzelschocks von je 2,1 mAh abgeben (≈120 mAh). Es verbleibt dann in der zweiten Batterie 2 eine Kapazität von ≈230 mAh, genug für 16 Monate Schrittmacherbetrieb und zusätzlich 16 Einzelschocks mit je 2,1 mAh. Somit ergibt sich eine Mindestbetriebsdauer für den Defibrillator D mit der Batterieanordnung 1, 2 (Hybridbatterie) von 6,2 Jahren.

Wie aus Figur 1 ersichtlich, sind beide Batterien an die Eingänge eines Stromversorgungsteils 3 angeschlossen, dessen Ausgang eine Steuerschaltung 4 versorgt. Die zweite Batterie ist zusätzlich an eine Hochstromschaltung 5 angeschlossen. Diese enthält bei dem dargestellten implantierbaren Defibrillator Schaltungen zur Aufladung der Schockkondensatoren und zur Schockabgabe (in der Figur nicht dargestellt). Die Steuerung 6 hierfür ist - neben einer an sich bekannten Herzschrittmacherschaltung 7 - Bestandteil der Steuerschaltung 4. Der Stromversorgungsteil 3 besteht im wesentlichen aus einem zur ersten Batterie 1 parallel geschalteten Kondensator 8, einem Spannungskomparator 9, einem Logikteil 10, zwei elektronischen Schaltern 11 und 12 sowie einem Spannungsregler 13. Die elektronischen Bauelemente, welche nach einem entsprechenden Spannungsabfall die Umschaltung der Versorgung der Schrittmacherschaltung in Bezug auf die Batteriekomponenten veranlassen sind in dem Block 3.1 zusammengefaßt.

In Figur 1 ist durch den Pfeil 22 angedeutet, daß mit der Betätigung der Schalter 11 und 12 ein zusätzliches Schaltsignal an den Spannungsregler abgegeben wird. Dieser ist in diesem Fall als ein eine Ladungspumpe aufweisender Spannungswandler ausgestaltet. Durch eine entsprechende Heraufsetzung der an der Spannungswandlung beteiligten Schaltstufen bzw. eine Verringerung der Anzahl der Taktzyklen bei fester Stufenzahl, wird die Stromversorgung des Schrittmacherteils an die höhere Spannung der Defibrillatorbatterie angepaßt. Auf diese Weise kann verhindert werden, daß beim Übergang auf eine höhere Versorgungsspannung sich der Wirkungsgrad der Schaltung dadurch verschlechtert, daß Energie in Wärme umgesetzt wird. Entsprechende Ladungspumpenschaltungen sind dem Fachmann bekannt.

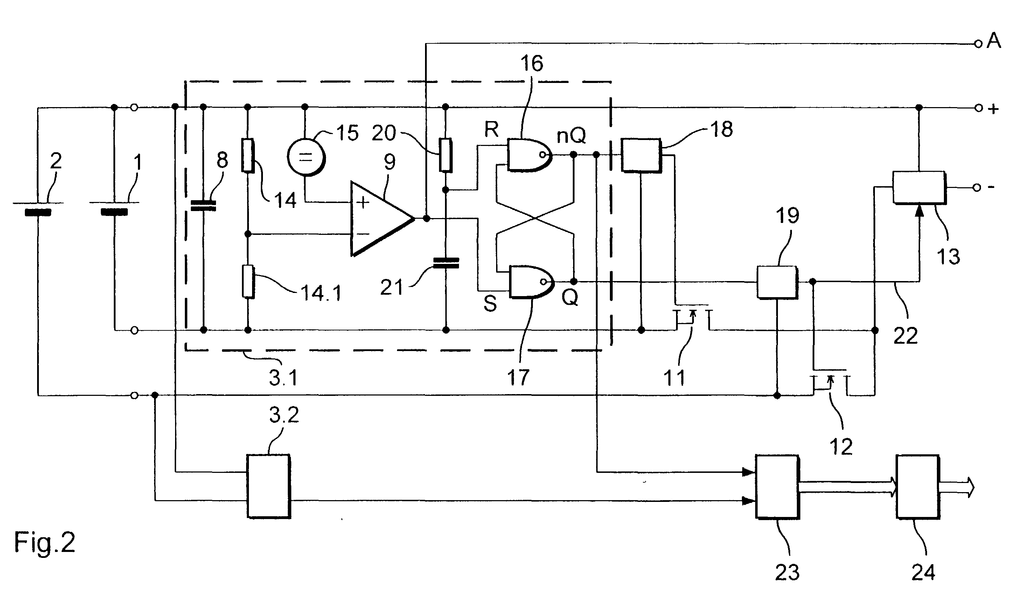

Wie aus Figur 2 ersichtlich, wird im Spannungskomparator 9 die mittels eines Spannungsteilers 14 geteilte Spannung der ersten Batterie 1 mit der Spannung einer Referenzspannungsquelle 15 verglichen. Das das Ergebnis des Vergleichs widerspiegelnde Ausgangssignal des Spannungskomparators 9 gelangt sowohl an einen Ausgang A - und steht dort der Steuerschaltung zur Auswertung (beispielsweise zur ERI-Bestimmung) zur Verfügung - als auch an den Setz-Eingang eines aus zwei NAND-Gattern 16 und 17 aufgebauten Flipflops. Das Flipflop befindet sich nach dem Anschließen der Batterie 1 zunächst im Rücksetz-Zustand, was durch ein aus einem Widerstand 20 und einem Kondensator 21 bestehendes RC-Glied bewirkt ist. Über einen Ausgang nQ und eine Pegelschieberschaltung (Levelshifter) 18 ist ein als elektronischer Schalter wirkender Transistor 11 eingeschaltet, d. h. der Eingang des Spannungsreglers 13 ist mit der ersten Batterie 1 verbunden. Andererseits ist der als Transistorschalter ausgeführte elektronische Schalter 12, der über den Q-Ausgang des Flipflop 16, 17 und einen weiteren Levelshifter 19 angesteuert wird, gesperrt.

Sinkt die Spannung der ersten Batterie 1 unter einen vorgegebenen Wert, der mittels der Referenzspannungsquelle 15 und des aus den beiden Widerständen 14 und 14.1 bestehenden Spannungsteilers festgelegt wird, schaltet der Komparator 9 und das aus den beiden über Kreuz verbundenen NAND-Gattern 16 und 17 aufgebaute Flipflop wird gesetzt. Folglich werden der Transistor 12 eingeschaltet und der Transistor 11 gesperrt, und der Eingang des Spannungsreglers 13 ist mit der zweiten Batterie 2 verbunden. Die Schaltung bleibt auch nach Erholung der ersten Batterie 1 in diesem Zustand. Die Steuerschaltung wird in der Folge ausschließlich durch die zweite Batterie 2 versorgt.

Bei einer modifizierten Ausführung, bei der die Schaltung nach dem Wiederansteigen der Spannung der ersten Batterie 1 wieder in den Ausgangszustand zurückkippen soll, ist das Flipflop weggelassen, und der Spannungskomparator 9 ist mit einer entsprechenden Schalthysterese versehen. In dieser Ausführung liefert die erste Batterie 1 trotz weitgehender Erschöpfung nach Erholung jeweils intermittierend weiterhin Beiträge zur Stromversorgung der Steuerschaltung. Dadurch läßt sich die Enrergie der ersten Batteriekomponente auch dann noch ausnutzen, wenn sie allein nicht mehr eine zur Aufrechterhaltung des Bedarfs der ersten Therapiekomponente ausreichenden Energie liefern könnte.

Der Kondensator 8 in der Baugruppe 3.1 verhindert durch seine Pufferfunktion ein starkes Zusammenbrechen der Spannung der ersten Batterie 1 bei kurzzeitigen Stromspitzen, wie sie vorzugsweise bei der Abgabe von Stimulationsimpulsen durch die Herzschrittmacherschaltung 7 auftreten. Mittels des Spannungsreglers 13 wird der nachfolgenden Steuerschaltung 4 eine konstante Spannung zur Verfügung gestellt. Der Spannungskomparator 9, die Referenzquelle 15 sowie die Gatter 16 und 17 werden ebenfalls mit dieser geregelten Spannung versorgt.

In Figur 2 ist zusätzlich zu der in Figur 1 dargestellten Ausführung noch eine Ergänzung vorgesehen. Hier wird nicht nur das Ausgangssignal des Spannungskomparators der Herzschrittmacherschaltung zur Verfügung gestellt, um eine End-Of-Life-Indikation auszulösen, sondern es ist zusätzlich vorgesehen, daß ein entsprechendes Signal auch telemetriert wird, um dem Arzt anzuzeigen, daß die normale Betriebsdauer des Schrittmachers erschöpft ist und nunmehr auf eine Art Reserve-Betrieb umgeschaltet wurde. Hierzu wird das Ausgangssignal des Flipflops 16, 17 einer Kodierschaltung 23 zugeführt, welcher das entsprechend verschlüsselte Signal einer Telemetrie-Schaltung 24, welche es als Sender an einen - nicht dargestellten - externen Empfänger übermittelt.

Zusätzlich ist noch eine weitere Baugruppe 3.2 vorgesehen, welche im wesentlichen der bereits beschriebenen Baugruppe 3.2 entspricht. Da auch die darin verwendeten Bauelemente übereinstimmen, konnte auf ein nähere Darstellung verzichtet werden. Mit der Baugruppe 3.2 wird die Restenergie der Batterie 2 entsprechend überwacht und rechtzeitig deren bevorstehende Erschöpfung anzeigendes Signal der Kodierschaltung 23 zugeführt, welches ebenfalls in dem verschlüsselten Signal als Information ausgegeben wird. Auf diese Weise kann der Arzt von außen die unterschiedlichen Stadien der Batterieerschöpfung erkennen und die entsprechenden Vorsorgemaßnahmen treffen. Wenn bei einem Patienten, welcher nur selten einen Defibrillationsschock erhalten muß, angezeigt wird, daß die Versorgung der Schrittmacherschaltung von der Batterie 1 auf die Batterie 2 umgeschaltet wurde, steht noch ausreichend Reservezeit zur Verfügung. Erst wenn auch noch die Baugruppe 3.2 ein Signal abgibt, muß eine Reimplantation geplant werden.

Die Anpassung des Übersetzungsverhältnisses der Spannungswandlerschaltung 13 über die Leitung 22 wurde schon anhand von Figur 1 beschrieben.

Die Auslegung der Energiequellen kann selbstverständlich - je nach konkretem Einsatzfall - auch in anderen Relationen erfolgen, vorzugsweise 6 Jahre bis zum Umschaltpunkt und 6 Monate bis zum Zeitpunkt EOL (End Of Life).

Die Erfindung beschränkt sich in ihrer Ausführung nicht auf das vorstehend angegebene bevorzugte Ausführungsbeispiel. Vielmehr ist eine Anzahl von Varianten günstig, welche von der dargestellten Lösung auch bei grundsätzlich anders gearteten Ausführungen Gebrauch macht.

## Patentansprüche

1. Batteriebetriebenes implantierbares medizinisches Mehrfunktionsgerät (PMD), welches eine erste Therapie-Baugruppe (7) mit niedrigem Strombedarf und eine zweite Therapie-Baugruppe (5) mit hohem Strombedarf sowie eine erste, an einen Verbraucher mit niedrigem Strombedarf angepaßten Batteriekomponente (1) und eine zweite, an einen Verbraucher mit hohem Strombedarf angepaßte Batteriekomponente (2), aufweist, insbesondere als Herzschrittmacher/Defibrillator-Kombinationsgerät, sowie
einen Fühler (14) zur Erfassung einer für die Restenergie der ersten Batteriekomponente charakteristischen Meßgröße, eine eingangsseitig mit dem Fühler (14) verbundene Meßeinrichtung zur Ausgabe eines Steuersignals, wenn die Restenergie der ersten Batteriekomponente für den Betrieb der ersten Therapie-Baugruppe mit niedrigem Strombedarf nicht mehr ausreichend ist, sowie
eine mit dem Ausgang der Meßeinrichtung verbundenen Schalteinrichtung (10 bis 13), **dadurch gekennzeichnet, daß** die Schalteinrichtung die zweite Batteriekomponente (2) bis zur Ausgabe des Steuersignals, welches anzeigt, daß eine vorbestimmte Restenergie der ersten Batteriekomponente unterschritten ist, nur mit der zweiten Therapie-Baugruppe (5), jedoch ab diesem Zeitpunkt mit der ersten Therapie-Baugruppe (7) verbindet und die erste Batteriekomponente (1) bei Verbindung der zweiten Batteriekomponente (2) mit der ersten Therapie-Baugruppe (7) von der ersten Therapie-Baugruppe trennt.

2. Mehrfunktionsgerät nach Anspruch 1, **dadurch gekennzeichnet, daß** es sich bei der für die Restenergie der ersten Batteriekomponente charakteristischen Meßgröße um deren Klemmenspannung oder deren Innenwiderstand handelt.

3. Mehrfunktionsgerät nach Anspruch 1 oder 2, **dadurch gekennzeichnet, daß** es sich bei der Meßeinrichtung zur Erfassung einer für die Restenergie unter Berücksichtigung des Strom- und/oder Spannungsbedarfs der ersten Batteriekomponente charakteristischen Meßgröße um eine Vergleicherschaltung (9) handelt, welche die Meßgröße mit einer Bezugsgröße vergleicht und ein Ausgangssignal abgibt, wenn zwischen Meßgröße und Vergleichsgröße eine vorgegebene Abweichung überschritten ist.

4. Mehrfunktionsgerät nach Anspruch 3, **dadurch gekennzeichnet, daß** es sich bei der Meßgröße um die Klemmenspannung und bei der Vergleichsgröße um eine Referenzspannung handelt.

5. Mehrfunktionsgerät nach Anspruch 1, **dadurch gekennzeichnet, daß** die Schalteinrichtung die zweite Batteriekomponente (2) nach Verbindung mit der ersten Therapie-Baugruppe (7) auch mit der zweiten Therapie-Baugruppe (5) verbunden hält.

6. Mehrfunktionsgerät nach einem der vorangehenden Ansprüche, **dadurch gekennzeichnet, daß** die erste und die zweite Batteriekomponente (1, 2) jeweils eine hermetisch abgeschlossene Batterie auf Lithiumbasis ist.

7. Mehrfunktionsgerät nach einem der vorangehenden Ansprüche, **dadurch gekennzeichnet, daß** die erste Batteriekomponente (1) eine Lithium/Iod-Batterie hoher Energiedichte und die zweite Batteriekomponente (2) eine Lithium/Braunstein-Batterie oder eine Lithium-Penta-Vanadin-Batterie hoher Leistungsdichte ist.

8. Mehrfunktionsgerät nach Anspruch 8, **dadurch gekennzeichnet, daß** das Kathodenmaterial der zweiten Batteriekomponente (2) aus einer Mischung von Braunstein mit anderen Metalloxiden besteht.

9. Mehrfunktionsgerät nach einem der vorangehenden Ansprüche, **dadurch gekennzeichnet, daß** zur Stromversorgung der ersten Therapie-Baugruppe (7) eine Niedrigstrom-Steuerschaltung (4) vorgesehen und die zweite Therapie-Baugruppe (5) über Steuerleitungen mit der Niedrigstrom-Steuerschaltung verbunden ist.

10. Mehrfunktionsgerät nach einem der vorangehenden Ansprüche, **dadurch gekennzeichnet, daß** die Vergleichereinheit (9) oder ein dieser nachgeordneter Logikteil (10) eine Hysterese-Charakteristik aufweist derart, daß die erste Batteriekomponente (1) bei einem Wiederanstieg der Klemmenspannung oder einem Wiederabsinken des Innenwiderstandes erneut mit der ersten Therapie-Baugruppe (7) verbunden wird.

11. Mehrfunktionsgerät nach einem der vorangehenden Ansprüche, **dadurch gekennzeichnet, daß** die Schalteinrichtung einen mit dem Ausgang der Vergleichereinheit (9) verbundenen Logikteil (10), einen ersten und zweiten über den Logikteil angesteuerten elektronischen Schalter (11, 12) und einen über die elektronischen Schalter eingangsseitig wahlweise mit der ersten oder zweiten Batteriekomponente verbindbaren Spannungsregler oder -wandler (13) aufweist.

12. Mehrfunktionsgerät nach Anspruch 11 bei dem die erste und die zweite Batteriekomponente eine unterschiedliche Klemmenspannung aufweisen, **dadurch gekennzeichnet, daß** der Spannungswandler (13) durch das Steuersignal in seinem Übersetzungsverhältnis derart umschaltbar ist, daß es bei Verbindung der ersten Therapie-Baugruppe (7) mit der ersten Batteriekomponente im wesentlichen dem Verhältnis der Klemmenspannung der ersten Batteriekomponente (1) zur Versorgungsspannung der ersten Therapie-Baugruppe (7) und bei bei Verbindung der ersten Therapiebaugruppe (7) mit der zweiten Batteriekomponente (2) im wesentlichen dem Verhältnis der Klemmenspannung der zweiten Batteriekomponente (2) zur Versorgungsspannung der ersten Therapiebaugruppe (7) entspricht.

13. Mehrfunktionsgerät nach Anspruch 3 und 9, **dadurch gekennzeichnet, daß** der Ausgang der Vergleichereinheit (9) oder der Meßeinrichtung mit einem Eingang der Niedrigstrom-Steuerschaltung (4) verbunden ist.

14. Mehrfunktionsgerät nach einem der Ansprüche 7 bis 14, **gekennzeichnet durch**
einen weiteren Fühler mit einer weiteren Meßeinrichtung zur Erfassung einer für die Restenergie der zweiten Batteriekomponente unter Berücksichtigung des Strom- und/oder Spannungsbedarfs der ersten Therapiebaugruppe charakteristischen Meßgröße,
eine eingangsseitig mit dem weiteren Fühler verbundenen Meßeinrichtung zur Ausgabe eines weiteren Steuersignals, wenn die Restenergie der zweiten Batteriekomponente für den Betrieb der ersten Therapie-Baugruppe mit niedrigem Strombedarf nur noch für einen begrenzten Zeitraum ausreichend ist, sowie
eine mit dem Ausgang der weiteren Meßeinrichtung verbundene Telemetrieeinrichtung (24), die ein Telemetriesignal an einen außerhalb des Körpers des Patienten befindlichen Empfänger abgibt, welches von dem weiteren Steuersignal beeinflußt ist.

15. Mehrfunktionsgerät nach Anspruch 17, **gekennzeichnet durch** eine Codiereinrichtung, welche das Ausgangssignal der weiteren Meßeinrichtung mit demjenigen der Meßeinrichtung derart zusammenführt, daß das Telemetriesignal sowohl von dem Steuersignal als auch von dem weiteren Steuersignal in unterschiedlicher Weise beeinflußt ist.

## Claims

1. A battery-operated, implantable multi-function medical device (PMD), which comprises a first therapy module (7) having a low current requirement and a second therapy module (5) having a large current requirement, and also a first battery component (1) adapted to a consumer having a low current requirement and a second battery component (2) adapted to a consumer having a high current requirement, in particular as a cardiac pacemaker/defibrillator combination device, and also
a sensor (14) for recording a measured variable characteristic of the residual energy of the first battery component,
a measurement instrument connected at the input side to the sensor (14) for the output of a control signal when the residual energy of the first battery component is no longer adequate for the operation of the first therapy module having a low current requirement, and also
a switching device (10 to 13) connected to the output of the measuring device,
**characterised in that** the switching device connects the second battery component (2) only to the second therapy module (5) until the control signal is outputted, which indicates that a predetermined residual energy of the first battery component is not met, but from this time connects it with the first therapy module (7) and separates the first battery component (1) from the first therapy module when the second battery component (2) is connected to the first therapy module (7).

2. A multi-function device according to Claim 1,
**characterised in that** the measured variable characteristic of the residual energy of the first battery component is its terminal voltage or its internal resistance.

3. A multi-function device according to Claim 1 or 2,
**characterised in that** the measuring device for recording a measured variable characteristic for the residual energy taking into consideration the current and/or voltage requirement of the first battery component is a comparator circuit (9), which compares the measured variable with a reference variable and emits an output signal when a predetermined deviation between the measurement variable and comparison variable is exceeded.

4. A multi-function device according to Claim 3,
**characterised in that** the measured variable is the terminal voltage and the comparison variable is a reference voltage.

5. A multi-function device according to Claim 1,
**characterised in that** the switching device keeps the second battery component (2) also connected with the second therapy module (5) after connection with the first therapy module (7).

6. A multi-function device according to one of the preceding Claims,
**characterised in that** the first and the second battery component (1, 2) are in each case a hermetically sealed battery on a lithium base.

7. A multi-function device according to one of the preceding Claims,
**characterised in that** the first battery component (1) is a lithium/iodine battery having a high energy density and the second battery component (2) is a lithium/manganese dioxide battery or a lithium penta-vanadium battery having a high power density.

8. A multi-function device according to Claim 8,
**characterised in that** the cathode material of the second battery component (2) consists of a mixture of manganese dioxide and other metal oxides.

9. A multi-function device according to one of the preceding Claims,
**characterised in that** a low-current control circuit (4) is provided for the current supply of the first therapy module (7) and the second therapy module (5) is connected via control lines to the low-current control circuit.

10. A multi-function device according to one of the preceding Claims,
**characterised in that** the comparator unit (9) or a logic component (10) connected downstream therefrom comprises a hysteresis characteristic such that the first battery component (1) is re-connected to the first therapy module (7) when the terminal voltage increases again or the internal resistance drops again.

11. A multi-function device according to one of the preceding Claims,
**characterised in that** the switching device comprises a logic component (10) connected to the output of the comparator unit (9), a first and second electronic switch (11, 12) controlled via the logic component and a voltage regulator or converter (13) which can optionally connected on the input side to the first or second battery component via the electronic switches.

12. A multi-function device according to Claim 11, in which the first and the second battery components have different terminal voltages,
**characterised in that** the voltage converter (13) can be switched by means of the control signal with respect to its transmission coefficient in such a manner that when the first therapy module (7) is connected to the first battery component it corresponds essentially to the ratio of the terminal voltage of the first battery component (1) to the supply voltage of the first therapy module (7) and when the first therapy module (7) is connected to the second battery component (2) corresponds essentially to the ratio of the terminal voltage of the second battery component (2) to the supply voltage of the first therapy module (7).

13. A multi-function device according to Claim 3 and 9,
**characterised in that** the output of the comparator unit (9) or of the measuring device is connected to an input of the low-current control circuit (4).

14. A multi-function device according to one of Claims 7 to 14,
**characterised by**
a further sensor having a further measuring device for recording a measured variable that is characteristic of the residual energy of the second battery component, taking into consideration of the current and/or voltage requirement of the first therapy module,
a measuring device, connected at the input side to the further sensor, for the emission of a further control signal when the residual energy of the second battery component for the operation of the first therapy module having a low current requirement is only sufficient for a limited time, and also
a telemetry device (24) connected to the output of the further measuring device, which transmits a telemetry signal to a receiver located outside the patient's body, the signal being influenced by the further control signal.

15. A multi-function device according to Claim 17,
**characterised by** an encoding device, which combines the output signal of the further measuring device with the output signal of the measuring device in such a manner that the telemetry signal is influenced both by the control signal and also by the further control signal in different ways.

## Revendications

1. Appareil médical implantable multifonctionnel (PMD) alimenté par pile, qui comporte un premier module de thérapie (7) avec un faible besoin en courant et un second module de thérapie (5) avec un besoin élevé en courant, ainsi qu'un premier composant formant pile (1) adapté à un appareil d'utilisation ayant un faible besoin en courant, et un second composant formant pile (2) adapté à un appareil d'utilisation présentant un besoin élevé en courant, notamment un appareil combiné formant stimulateur cardiaque et/ou défibrillateur, et
un capteur (14) pour détecter une grandeur de mesure caractéristique de l'énergie résiduelle du premier composant formant pile,
un dispositif de mesure relié côté entrée au capteur (14) et servant à délivrer un signal de commande lorsque l'énergie résiduelle du premier composant formant pile n'est plus suffisante pour un fonctionnement du premier module de thérapie présentant un faible besoin en courant, et
un dispositif de commutation (10 à 13), qui est relié à la sortie du dispositif de mesure,
**caractérisé en ce que** le dispositif de commutation relie le second composant formant pile (2) uniquement au second module de thérapie (5) jusqu'à la délivrance du signal de commande qui indique que l'énergie du premier composant formant pile est tombée au-dessous d'une énergie résiduelle prédéterminée, , mais le relie, à partir de cet instant, au premier module de thérapie (4) et sépare le premier composant formant pile (1) du premier module de thérapie lors de la liaison du second module formant pile (2) au premier module de thérapie (7).

2. Appareil multifonctionnel selon la revendication 1, **caractérisé en ce qu'**en ce qui concerne la grandeur de mesure caractéristique de l'énergie résiduelle du premier composant formant pile, il s'agit de sa tension aux bornes ou de sa résistance intérieure.

3. Appareil multifonctionnel selon la revendication 1 ou 2, **caractérisé en ce qu'**en ce qui concerne le dispositif de mesure pour détecter une grandeur de mesure caractéristique en prenant en compte le besoin en courant et/ou le besoin en tension du premier composant formant pile, il s'agit d'un circuit comparateur (9) qui compare la grandeur de mesure à une grandeur de référence et délivre un signal de sortie lorsqu'un écart prédéterminé est dépassé entre la grandeur de mesure et la grandeur de référence.

4. Appareil multifonctionnel selon la revendication 3, **caractérisé en ce qu'**en ce qui concerne la grandeur de mesure il s'agit de la tension aux bornes et qu'en ce qui concerne la grandeur de référence il s'agit d'une tension de référence.

5. Appareil multifonctionnel selon la revendication 1, **caractérisé en ce qu'**après la liaison avec le premier module de thérapie (7), le module de commutation maintient le second composant formant pile (2) également relié au second module de thérapie (5).

6. Appareil multifonctionnel selon l'une des revendications précédentes, **caractérisé en ce que** les premier et second composants formant piles (1,2) sont chacun une pile à base de lithium fermée hermétiquement.

7. Appareil multifonctionnel selon l'une des revendications précédentes, **caractérisé en ce que** le premier composant formant pile (1) est une pile à lithium/iode à haute densité d'énergie et que le second composant formant pile (2) est une pile à lithium/bioxyde de manganèse ou une pile lithium-penta-vanadium ayant une haute densité de puissance.

8. Appareil multifonctionnel selon la revendication 7, **caractérisé en ce que** le matériau de cathode du second composant formant pile (2) est constitué par un mélange de dioxyde de manganèse et d'autres oxydes métalliques.

9. Appareil multifonctionnel selon l'une des revendications précédentes, **caractérisé en ce que** pour l'alimentation en courant du premier module de thérapie (7), il est prévu un circuit (4) de commande à courant faible et que le second module de thérapie (5) est relié au moyen de lignes de commande au circuit de commande de courant faible.

10. Appareil multifonctionnel selon l'une des revendications précédentes, **caractérisé en ce que** l'unité de comparaison (9) ou une partie logique (10) branchée en aval de cette unité, possède une caractéristique d'hystérésis de telle sorte que le premier composant formant pile (1) est à nouveau relié au premier module de thérapie (7) lors de la remontée de la tension aux bornes ou d'une nouvelle baisse de la résistance interne.

11. Appareil multifonctionnel selon l'une des revendications précédentes, **caractérisé en ce que** le dispositif de commutation comporte une partie logique (10) reliée à la sortie de l'unité formant comparateur (9), des premier et second interrupteurs électroniques (11,12) commandés par l'intermédiaire de la partie logique, et un régulateur ou convertisseur de tension (13) pouvant être relié côté entrée, par l'intermédiaire des interrupteurs électroniques, au choix au premier composant formant pile ou au second composant formant pile.

12. Appareil multifonctionnel selon la revendication 11, dans lequel le premier composant formant pile et le second composant formant pile possèdent des tensions aux bornes différentes, **caractérisé en ce que** le rapport de démultiplication du convertisseur de tension (13) peut être commuté par le signal de commande de telle sorte que lors de la liaison du premier module de thérapie (7) avec le premier composant formant pile, ce rapport correspond essentiellement au rapport de la tension aux bornes du premier composant formant pile (1) à la tension d'alimentation du premier module de thérapie (7) et, dans le cas de la liaison du premier module de thérapie (7) au second composant formant pile (2), essentiellement au rapport de la tension aux bornes du premier composant formant pile (2) à la tension d'alimentation du premier composant de thérapie (7).

13. Appareil multifonctionnel selon les revendications 3 et 9, **caractérisé en ce que** la sortie de l'unité formant comparateur (9) ou du dispositif de mesure est reliée à une entrée du circuit (4) de commande à courant faible.

14. Appareil multifonctionnel selon l'une des revendications 7 à 14, **caractérisé par**
un autre capteur comportant un autre dispositif de mesure pour détecter une grandeur de mesure caractéristique de l'énergie résiduelle du second composant formant pile en tenant compte du besoin en courant et/ou du besoin en tension du premier module de thérapie, un dispositif de mesure relié côté entrée à l'autre capteur et servant à délivrer un autre signal de commande lorsque l'énergie résiduelle du second composant formant pile est seulement encore suffisante pour un intervalle de temps limité pour le fonctionnement du premier module de thérapie présentant un faible besoin en courant, et
un dispositif de télémétrie (24), qui est relié à la sortie de l'autre dispositif de mesure et délivre à un récepteur situé à l'extérieur du corps du patient, un signal de télémétrie, qui est influencé par l'autre signal de commande.

15. Appareil multifonctionnel selon la revendication 17, **caractérisé par** un dispositif de codage, qui envoie le signal de sortie de l'autre dispositif de mesure de telle sorte que le signal de télémétrie est influencé de façon différente aussi bien par le signal de commande que par l'autre signal de commande.
